# EUROPÄISCHE PATENTSCHRIFT

(11) **EP 2 277 573 B1**
(45) Veröffentlichungstag und Bekanntmachung des Hinweises auf die Patenterteilung: **14.08.2019**
(21) Anmeldenummer: 10167722.7
(22) Anmeldetag: 29.06.2010
(51) Int. Cl.: A61M 1/34, A61M 1/02

(54) **Verfahren zur gravitations-effizierten Plasmapherese**
Method for gravitation-caused plasma pheresis
Procédé de plasmaphérèse efficace par gravitation

(30) Priorität: 03.07.2009 DE 102009033071
(43) Veröffentlichungstag der Anmeldung: 26.01.2011
(73) Patentinhaber: Lmb Lab med Blutbank Technologie GmbH, 85445 Schwaig (DE)
(72) Erfinder: Stute, Reinhard, 66121, Saarbrücken (DE); Jentsch, Klaus, 85445, Schwaig (DE)
(74) Vertreter: Winter, Brandl, Fürniss, Hübner, Röss, Kaiser, Polte - Partnerschaft mbB

(56) Entgegenhaltungen:
- EP-A1- 2 255 838
- WO-A1-00/62840
- DE-A1- 10 256 848

## Beschreibung

Die vorliegende Erfindung betrifft eine Vorrichtung zum Ausführen einer Plasmapherese und insbesondere einer gravitations-effizierten Plasmapherese gemäß Anspruch 1.

Unter einer Plasmapherese versteht man ein Verfahren zur Gewinnung von (Blut)plasma zu therapeutischen Zwecken (Plasma-Austausch) oder zur Plasmaspende. Dabei wird extrakorporal mit Hilfe einer Plasmapheresevorrichtung die Plasmafraktion vom Restblut, einem Konzentrat aus zellulären Blutbestandteilen, separiert. Die Entnahme des Plasmas muss hierbei isovolämisch durchgeführt werden, d. h. es muss konstant eine gleiche Menge an Substitutionslösung infundiert werden wie Plasmavolumen eliminiert wird.

Die extrakorporale Separation erfolgt herkömmlich mit Pumpen und Zentrifugen, wobei in der Regel in dem Sinne kein Kreislauf gebildet wird, dass einem Patienten / Spender in einem Blutentnahmebeutel eine definierte Blutmenge entnommen wird, der dann vom Patienten getrennt wird. Die Blutbestandteile des in dem Blutentnahmebeutel gesammelten Blutes werden dann in einer nicht mit dem Patienten verbundenen Zentrifuge auf der Grundlage ihrer unterschiedlichen Dichte separiert, und das auf diese Weise gewonnene Konzentrat aus zellulären Bestandteilen wird dem Patienten / Spender zurück transfundiert. Dieser Vorgang ist nicht nur mit einem hohen Kosten- und Geräteaufwand verbunden, sondern birgt durch das Abtrennen und Neuverbinden des Blutentnahmebeutels, dessen Transport und die Zahl involvierter Geräte und Personen auch viele Kontaminationsgefahren. Darüber hinaus dauert der gesamte Vorgang sehr lange.

Vorrichtungen zum Ausführen einer Plasmapherese sind im Stand der Technik beispielsweise aus der DE 102 56 848 A1 und der WO 00/62840 bekannt.

In der DE 102 56 848 A1 ist ein technisch einfaches Verfahren zum Separieren von Vollblut beschrieben, bei dem möglichst wenig Plasma in Erythrozyten-Konzentrat verbleibt. Nach einer ersten Filtration enthält der zweite Blutbeutel das gefilterte Blut und der Plasmabeutel das durch den Plasmafilter separierte Plasma. Das Blut aus dem zweiten Blutbeutel fließt in zur ersten Filtration umgekehrter Flussrichtung durch den Schlauch zwischen zweitem Blutbeutel und dem Plasmafilter durch den Plasmafilter und das gefilterte Blut fließt in den dritten Blutbeutel und das separierte Plasma in den Plasmabeutel.

In der WO 00/62840 ist ebenfalls eine Vorrichtung zum Auftrennen von Blut in einzelne und/oder Gruppen seiner Bestandteile offenbart. Diese bekannte Vorrichtung weißt dazu eine Filteranordnung, einen Aufnahmebhälter für Blutplasma und einen Aufnahmebehälter für zellulare Bestandteile des Blutes auf. Eine mehrfache Filtration nicht vorgesehen.

Im Gegensatz zur WO 00/62840 kann bei Einsatz der vorliegenden Erfindung eine mehrfache Filtration ausgeführt werden. Dazu weist die vorliegende Erfindung, im Gegensatz zur DE 102 56 848 A1, einen Rückflusspfad vom Konzentratbeutel zum Entnahmebeutel auf, wodurch die Anzahl der für die Plasmapherese benötigten Anzahl von Beuteln reduziert werden kann. Zudem können mehrere Filtrationsdurchläufe ausgeführt werden. Als ein weiterer Vorteil kann die Vorrichtung gemäß der vorliegenden Erfindung das Blut des Spenders der Vorrichtung ohne zusätzlichen Blutbehälter direkt von der Vene des Spenders zugeführt werden, da mittels der Vorrichtung Kochsalz in die Vene des Spenders infudiert werden kann, was bei einer isovolämisch durchgeführten Entnahme von Plasma erforderlich ist.

Es ist daher Aufgabe der vorliegenden Erfindung, die oben genannten Nachteile zu vermeiden und eine Vorrichtung zum Ausführen einer Plasmapherese bereitzustellen, die kostengünstig, einfach anzuwenden und patientenfreundlich ist und bei der die Kontaminationsgefahr minimiert ist.

Diese Aufgabe wird durch die Merkmale des Anspruchs 1 gelöst.

Eine Plasmapherese wird mit Hilfe einer mit einer Vene eines Patienten verbundenen Plasmapheresevorrichtung gemäß der vorliegenden Erfindung durchgeführt, die in einer Ausführungsform der vorliegenden Erfindung einen Entnahmebeutel zum Aufnehmen einer vorbestimmten Blutentnahmemenge des Patienten, ein Hohlfaserfilter zum Separieren der Blutentnahmemenge in ein Konzentrat aus zellulären Bestandteilen und Plasma, einen Plasmabeutel zum Aufnehmen des durch das Hohlfaserfilter separierten Plasmas und einen Konzentratbeutel zum Aufnehmen des durch das Hohlfaserfilter separierten Konzentrats aus zellulären Bestandteilen umfasst.

Aus der Tatsache, dass der erste Fließweg unterbrochen werden kann, folgt, dass die isotonische Kochsalzlösung nur oberhalb der Unterbrechungsstelle des ersten Fließweges infudiert wird, also zwischen dieser und einer Punktionsstelle einer Blutentnahmenadel, in den ersten Fließweg eingeleitet werden kann. Ferner folgt aus der Tatsache, dass die Infusion der isotonischen Kochsalzlösung dem Offenhalten der Vene dient, dass die isotonische Kochsalzlösung über die Punktionsstelle der Blutentnahmenadel infundiert wird, wodurch das Blut des Spenders der Vorrichtung ohne einen zusätzlichen Blutbeutel zugeführt werden kann.

Bei der Plasmapherese erfolgt der Transport des zunächst noch als Bestandteil der Blutabnahmemenge vorliegenden Plasmas von der Vene über den Blutentnahmebeutel, der während des gesamten Prozesses mit dem Patienten verbunden bleibt, und das Hohlfaserfilter, in dem es von den zellulären Bestandteilen separiert wird, bis zu dem Plasmabeutel gravitations-effiziert, indem der Entnahmebeutel zur Erzeugung eines hydrostatischen Drucks über dem Hohlfaserfilter angeordnet ist. Da hierbei der vertikale Abstand zwischen dem Entnahmebeutel und dem Hohlfaserfilter so eingestellt ist, dass bei gegebener Porengröße des Hohlfaserfilters dessen Filterwirkung optimal ist, kann auf einen maschinellen Transport über Pumpen oder dergleichen verzichtet werden; die treibende Kraft ist die Gravitation.

Die Verwendung des Hohlfaserfilters hat gegenüber anderen Filtertypen den Vorteil, dass durch die Anzahl, Auslegung (Wandstärke, Innendurchmesser, Länge, Porengröße, Materialien) und Packungsdichte der Fasern die Filtereigenschaften in weitem Umfang bedarfsadaptiv veränderbar sind. Darüber hinaus sind die Trennungseigenschaften gegenüber anderen Filtertypen besser, so dass eine Bestimmung des als Hämatokrit bezeichneten Anteils zellulären Blutbestandteile zum Gesamtblut damit möglich ist.

Gemäß einer vorteilhaften Ausgestaltung der vorliegenden Erfindung wird das Konzentrat aus zellulären Bestandteilen, das nach dem Hohlfaserfilter, d. h. am Ende des vierten Fließweges IV vorliegt, nicht direkt in den Blutentnahmebeutel zurückgeführt, sondern zunächst in einem Konzentratbeutel gesammelt, ebenso wie erfindungsgemäß das Plasma in dem Plasmabeutel gesammelt wird. Dies hat den Vorteil, dass das Konzentrat ebenso wie das Plasma auf diese Weise steril aufbewahrt seinem Zweck zugeführt werden kann, im Falle einer Spende also nicht mehr dem Patienten zurück transfundiert wird. Der Transport von dem Konzentratbeutel zu dem Blutentnahmebeutel erfolgt wiederum gravitations-effiziert.

Der entscheidende Vorteil des Verzichts auf jedwede Automatisierung kommt insbesondere beim Einsatz des erfindungsgemäßen Verfahrens in Entwicklungsländern zum Tragen.

Gemäß einer vorteilhaften Ausgestaltung der vorliegenden Erfindung kann eine Blutentnahme und eine nachfolgende Plasmafilterung n-mal ausgeführt werden, wobei n eine nicht-negative ganze Zahl ist, und anschließend eine mehrmalige Filtration ausgeführt. Bei n = 2 finden somit zum Beispiel 3 Blut- bzw. Plasmaentnahmen statt. Die gesamte Plasmamenge pro Sitzung ist gesetzlich auf 600 ml begrenzt. Vorteilhafterweise wird diese Menge durch 3 Entnahmen von je 450 ml Blut gewonnen. Die in diesem Fall zugeführte Menge an Citrat-Phosphat-Dextrose (CPD) - Lösung ist 63 ml.

Gemäß einer vorteilhaften Ausgestaltung der vorliegenden Erfindung weisen die Fließwege jeweils wenigstens einen Schlauchabschnitt auf, und das Herstellen und Unterbrechen der jeweiligen Fließwege erfolgt durch Abklemmen bzw. Lösen der Abklemmung des jeweiligen wenigstens einen Schlauchabschnitts. Die Abfolge von Schritten beim Ausführen einer Plasmapherese mittels der erfindungsgemäßen Vorrichtung kann somit manuell durch entsprechendes Abklemmen bzw. Lösen der Abklemmung gesteuert werden. Durch geeignete Abklemmvorrichtungen sowie deren Positionen, vorteilhafterweise an den Endabschnitten der Schlauchverbindungen, lässt sich vorteilhafterweise jede einzelne Komponente der Plasmapheresevorrichtung getrennt von den weiteren Komponenten sauber entfernen, austauschen, reinigen, reparieren, etc. Durch die strikte Abfolge von Herstellen und Unterbrechen der jeweiligen Fließwege ist die Plasmapherese problemlos von einer einzigen Person ausführbar.

Die Eigenschaften und Vorteile der vorliegenden Erfindung sind aus der nachfolgenden detaillierten Beschreibung, die unter Bezugnahme auf die beigefügten Zeichnungen erfolgt, deutlicher ersichtlich. In den Figuren sind:
Fign. 1a und 1b schematische Darstellungen einer Plasmapheresevorrichtung gemäß der oben erwähnten vorteilhaften Ausführungsform der vorliegenden Erfindung; und
Fign. 2 bis 6 verschiedene Schritte des mittels der vorteilhaften Ausführungsform nach Fig. 1 durchgeführten Plasmapherese .

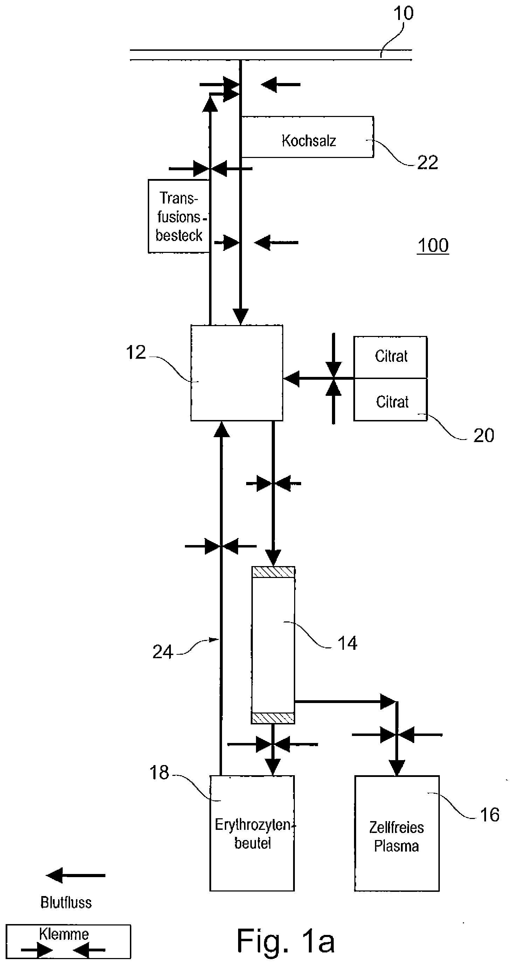

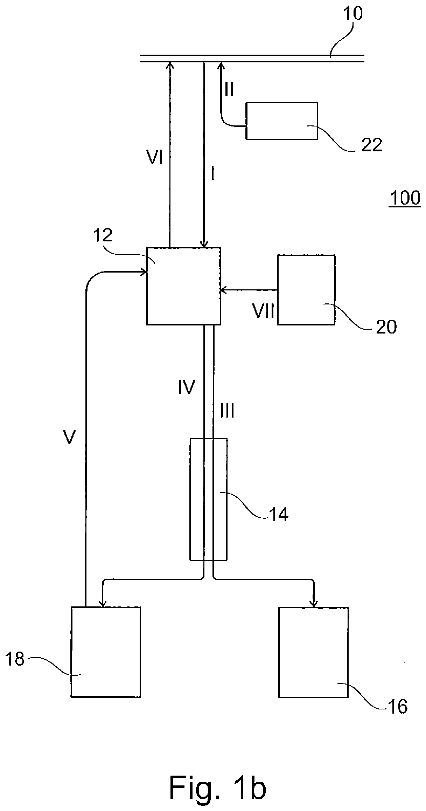

Fig. 1a zeigt eine schematische Plasmapheresevorrichtung 100. Die Plasmapheresevorrichtung 100 ist mit einer Vene 10 verbunden und umfasst einen Entnahmebeutel 12, ein Hohlfaserfilter 14, einen Plasmabeutel 16, einen Konzentratbeutel 18, einen CPD (Citrat-Phosphat-Dextrose) - Beutel 20 und einen Kochsalzlösungsbeutel 22. Die Komponenten 12-22 sind miteinander durch ein Schlauchsystem 24 verbunden, wobei durch Pfeile "→" zwischen den jeweiligen Komponenten 12-22 Schläuche, eine Verhinderung des Blutflusses durch einen jeweiligen Schlauch durch senkrecht zu den Schläuchen gezeichnete "geschlossene" Pfeile "→←" (geschlossene Klemme) und ein Erlauben des Blutflusses durch einen jeweiligen Schlauch durch senkrecht zu den Schläuchen gezeichnete "offene" Pfeile "→←" (offene Klemme) dargestellt sind. Blutwege, die gemäß der vorliegenden Erfindung wie es oben ausgeführt ist unterbrochen oder freigegeben werden, sind in Fig. 1b durch Pfeile "→" zwischen den jeweiligen Komponenten 12-22 eingezeichnet und mit römischen Ziffern bezeichnet. Insbesondere bezeichnet I. einen ersten Fließweg zwischen der Vene und dem Entnahmebeutel, II. einen zweiten Fließweg zwischen dem Kochsalzlösungsbeutel 20 und der Vene 10, III. einen dritten Fließweg zwischen dem Entnahmebeutel 12 und dem Plasmabeutel 16, IV: einen vierten Fließweg zwischen dem Entnahmebeutel 12 und dem Konzentratbeutel 18, V. einen fünften Fließweg von dem Konzentratbeutel 18 zu dem Entnahmebeutel 12, VI. einen sechsten Fließweg zwischen dem Entnahmebeutel 12 und der Vene 10 und VII. zwischen dem CPD-Beutel 20 und dem Blutentnahmebeutel 12.

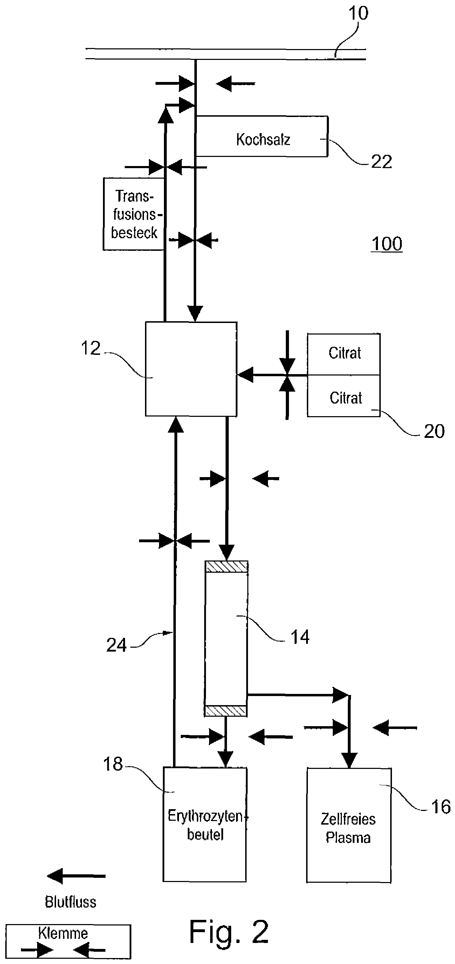

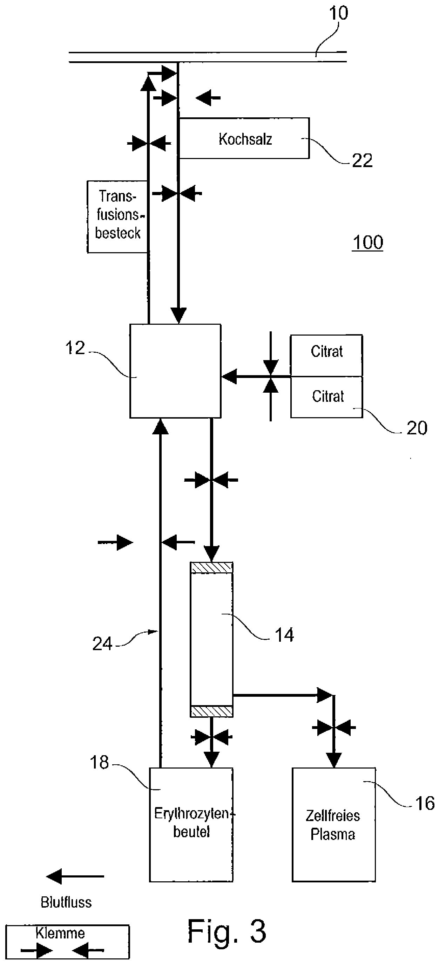

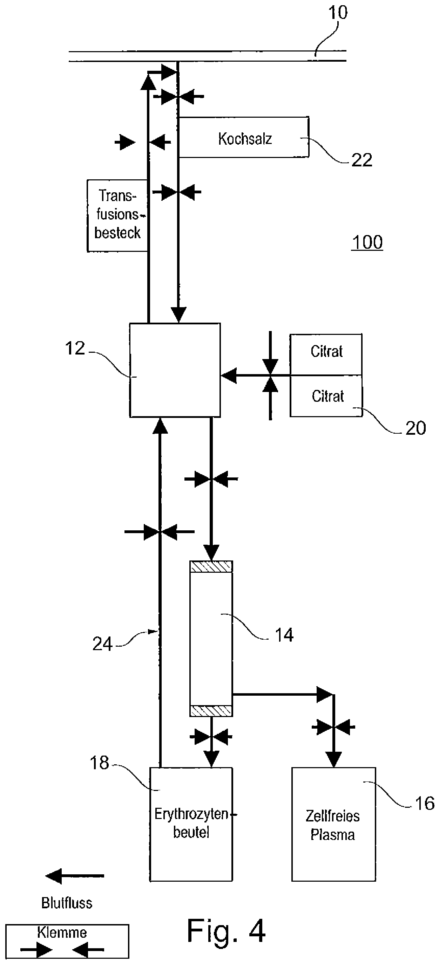

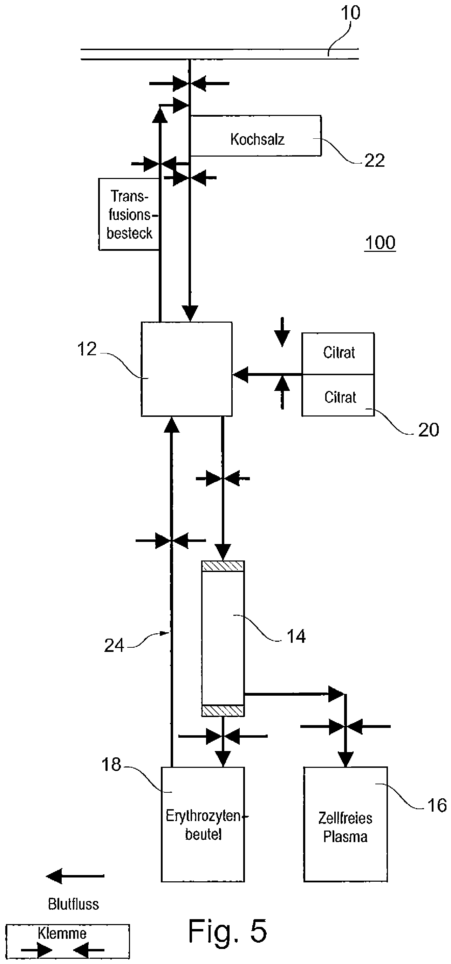

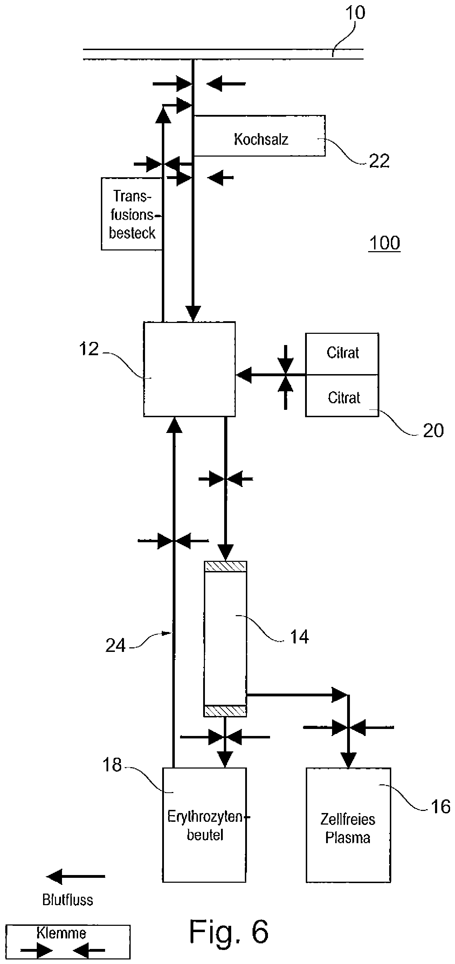

In den Fign. 1a/b bis 6 sind durch die Klemmen-Symbole "→←" und "→←" die Schritte der Plasmapherese entlang der Fließwege I. bis VII. dargestellt. Insbesondere ist zu beachten, dass auch über den Fließweg V. ein gravitations-effizierter Transport stattfindet, da die Beutel in den Figuren in ihrer räumlichen Anordnung nur schematisch dargestellt sind; die Positionen der Beutel sind nicht festgelegt, vielmehr können die Beutel entsprechend umgehängt werden. Wie es in den Fign. 1a/b bis 6 schematisch dargestellt ist, wird für die Infusion, Transfusion und Blutentnahme wohl bekanntes medizinisches Instrumentarium verwendet.

## Patentansprüche

1. Plasmapheresevorrichtung, mit:
einem Entnahmebeutel (12) zur Aufnahme einer vorbestimmten Menge Blutes,
einem ersten Fließweg (I), der mit dem Entnahmebeutel (12) verbunden ist und Blut an den Entnahmebeutel (12) liefert,
einem Beutel (20), der eine Citrat-Phosphat-Dextrose-Lösung enthält,
einem Beutel (22), der eine Kochsalzlösung enthält,
einem Hohlfaserfilter (14) zum Separieren des Blutes in ein Konzentrat aus zellulären Bestandteilen und Plasma,
einem Konzentratbeutel (18) zum Aufnehmen des durch das Hohlfaserfilter (14) separierten Konzentrats aus zellulären Bestandteilen,
einem Plasmabeutel (16) zum Aufnehmen des durch das Hohlfaserfilter (14) separierten Plasmas,
einem zweiten Fließweg (II), der mit dem die Kochsalzlösung enthaltenden Beutel (22) verbunden und dazu ausgelegt ist, mit einer Vene verbunden zu werden,
einem dritten Fließweg (III), der den Entnahmebeutel (12) mit dem Plasmabeutel (16) über das Hohlfaserfilter (14) verbindet und durch den das durch das Hohlfaserfilter (14) separierte Plasma in den Plasmabeutel (16) fließt,
einem vierten Fließweg (IV), der den Entnahmebeutel (12) und den Konzentratbeutel (18) über das Hohlfaserfilter (14) verbindet und durch den die durch das Hohlfaserfilter (14) konzentrierten zellulären Bestandteile in den Konzentratbeutel (18) fließen,
einem fünften Fließweg (V), der den Konzentratbeutel (18) direkt mit dem Entnahmebeutel (12) verbindet und durch den die durch das Hohlfaserfilter (14) konzentrierten zellulären Bestandteile von dem Konzentratbeutel (18) zurück in den Entnahmebeutel (12) fließen,
einem sechsten Fließweg (VI), der mit dem Entnahmebeutel (12) verbunden und dazu ausgelegt ist, mit einer Vene verbunden zu werden, und
einem siebten Fließweg (VII), der den die Citrat-Phosphat-Dextrose-Lösung enthaltenden Beutel (20) mit dem Blutentnahmebeutel (12) verbindet,
wobei der Entnahmebeutel (12) zur Erzeugung eines hydrostatischen Drucks in einer an die Porengröße des Hohlfaserfilters (14) angepassten Höhe über dem Hohlfaserfilter (14) angeordnet ist, und
wobei jeder Fließweg als Schlauchabschnitt mit einer Klemme zum selektiven Öffnen und Schließen des Fließwegs ausgebildet ist.

## Claims

1. Plasmapheresis device, with:
a collection bag (12) for receiving a predetermined amount of blood,
a first flow path (I) connected to the collection bag (12) and supplying blood to the collection bag (12),
a bag (20) containing a citrate-phosphate-dextrose solution,
a bag (22) containing a saline solution,
a hollow fiber filter (14) for separating the blood into a concentrate of cellular components and plasma,
a concentrate bag (18) for receiving the concentrate of cellular constituents separated by the hollow fiber filter (14),
a plasma bag (16) for receiving the plasma separated by the hollow fiber filter (14),
a second flow path (II) connected to the bag (22) containing the saline solution and adapted to be connected to a vein,
a third flow path (III) which connects the removal bag (12) to the plasma bag (16) via the hollow fiber filter (14) and through which the plasma separated by the hollow fiber filter (14) flows into the plasma bag (16),
a fourth flow path (IV) connecting the collection bag (12) and the concentrate bag (18) via the hollow fiber filter (14) and through which the cellular constituents concentrated by the hollow fiber filter (14) flow into the concentrate bag (18),
a fifth flow path (V) connecting the concentrate bag (18) directly to the collection bag (12) and through which the cellular components concentrated by the hollow fiber filter (14) flow from the concentrate bag (18) back into the collection bag (12),
a sixth flow path (VI) connected to the removal bag (12) and adapted to be connected to a vein, and
a seventh flow path (VII) connecting the bag (20) containing the citrate-phosphate dextrose solution to the blood collection bag (12),
wherein, for generating a hydrostatic pressure, the removal bag (12) is arranged at a height above the hollow-fiber filter (14) adapted to the pore size of the hollow-fiber filter (14), and
wherein each flow path is formed as a hose portion having a clamp for selectively opening and closing the flow path.

## Revendications

1. Dispositif de plasmaphérèse, avec :
une poche de prélèvement (12) destinée à recevoir une quantité prédéterminée de sang,
un premier trajet d'écoulement (I), qui est relié à la poche de prélèvement (12) et qui fournit du sang à la poche de prélèvement (12),
une poche (20), qui contient une solution de citrate-phosphate-dextrose,
une poche (22), qui contient une solution de sel de cuisine,
un filtre à fibres creuses (14) destiné à la séparation du sang en un concentré de constituants cellulaires et un plasma,
une poche de concentré (18) destinée à recevoir le concentré, séparé par le filtre à fibres creuses (14), de constituants cellulaires,
une poche de plasma (16) destinée à recevoir le plasma séparé par le filtre à fibres creuses (14),
un deuxième trajet d'écoulement (II), qui est relié à la poche (22) contenant la solution de sel de cuisine et qui est conçu pour être relié à une veine,
un troisième trajet d'écoulement (III), qui relie la poche de prélèvement (12) à la poche de plasma (16) par l'intermédiaire du filtre à fibres creuses (14) et par lequel le plasma séparé par le filtre à fibres creuses (14) s'écoule dans la poche de plasma (16),
un quatrième trajet d'écoulement (IV), qui relie la poche de prélèvement (12) et la poche de concentré (18) par l'intermédiaire du filtre à fibres creuses (14) et par lequel les constituants cellulaires concentrés par le filtre à fibres creuses (14) s'écoulent dans la poche de concentré (18),
un cinquième trajet d'écoulement (V), qui relie la poche de concentré (18) directement à la poche de prélèvement (12) et par lequel les constituants cellulaires concentrés par le filtre à fibres creuses (14) retournent de la poche de concentré (18) à la poche de prélèvement (12),
un sixième trajet d'écoulement (VI), qui est relié à la poche de prélèvement (12) et qui est conçu pour être relié à une veine, et
un septième trajet d'écoulement (VII), qui relie la poche (20) contenant la solution de citrate-phosphate-dextrose à la poche de prélèvement de sang (12),
dans lequel la poche de prélèvement (12) est agencée au-dessus du filtre à fibres creuses (14) à une hauteur adaptée à la dimension des pores du filtre à fibres creuses (14) afin de produire une pression hydrostatique, et
dans lequel chaque trajet d'écoulement est réalisé comme un tronçon de tuyau avec une pince pour l'ouverture et la fermeture sélectives du trajet d'écoulement.
